# EUROPEAN PATENT APPLICATION

(11) **EP 2 216 408 A1**
(43) Date of publication of application: **11.08.2010**
(21) Application number: 08842388.4
(22) Date of filing: 24.10.2008
(51) Int. Cl.: C12N 15/54, C12N 15/82

(54) **METHOD FOR IMPROVING SALINITY TOLERANCE**

(30) Priority: 24.10.2007 ES 200702926
(71) Applicant: Navarro Aviño, Juan Pedro, 46002 Valencia (ES)
(72) Inventor: LÓPEZ MOYA, José Rafael, Valencia (ES); NAVARRO AVIÑO, Juan Pedro, 46002 Valencia (ES)
(74) Representative: Urizar Anasagasti, José Antonio
(86) International application number: PCT/ES2008/000662
(87) International publication number: WO 2009/053511

(57) **Abstract**

The invention relates to a specific method for improving the tolerance to salinity of living organisms and eliminating sodium (Na⁺) from water, soil, sludge or any other medium containing said element, using the isolated nucleic acid sequence that codes for phytochelatin synthase of *Nicotiana glauca.*

LIST OF SEQUENCES

<110> Universidad Politécnica de Valencia
<120> Method to improve tolerance to salinity
<130> 222/07

<160> 4

<170> PatentIng version 3.4

<210> SEQ ID NO 1
<211> 1506
<212> DNA
<213> Nicotiana glauca

<400>
atggcgatgg cgggtttata tcggcgagtt cttccgtgcc ctccggctgt tgatttcgct 60
tctaccgaag gaaagcaact ttttttggag gccatccaga atggaacaat ggaaggattt 120
ttcaagttga tctcttattt tcagacacag tctgaaccgg cctattgtgg gttggctagc 180
ctttccatgg tcttgaatgc ccttgctatt gatccaggaa gaaaatggaa aggtccttgg 240
agatggttcg atgaatctat gttggactgt tgtgagcctt tggagaaggt taaagctaaa 300
gggatctctt ttgggaaagt ggtatgtttg gctcactgtg caggagcgaa ggtggaagct 360
tttcgctcta atcatagtac tattgatgat tttcgtaaac aagtcatggc ctgcactact 420
agtgataatt gtcatctgat ctcatcatac catagaggcc tttttaaaca gacaggttcg 480
ggccactttt cacctattgg tggttatcac gcgggaaagg atatggcact gattctagat 540
gttgcgaggt ttaaataccc ccctcactgg gttcccctcc ctctcctttg ggaagccatg 600
aacacaattg atgaagcacc aggattacat aggggtttta tgctaatttc taagcttcac 660
cgagctcctg cactgctata taccctgagc tgtaaacatg agagttgggt cactatctca 720
aagcatttga tggatgatct tcctgtcctg ttaagtcctg agaatgtgaa gggcataaaa 780
gatgttctct cttctgttct ttcaaatcta ccttcaaatt ttgttgaatt cataaagtgg 840
atagcagaag ttcgaaggca agaggagaat ggtcaaaatt tgagtgacga ggagaaagga 900
aggctagcta tcaaggaaga ggtattgaaa caagtgcagg acactcctct ttataagcat 960
gtcacaagca ttttattttc agaaaattct gtctgccagt caaatgcagc atcagacagc 1020
agtttggcta atgttgctgc aagcatttgc tgccaaggag caggtctttt tgcaggaaga 1080
tctggttcat cggataggtt ttgctgtcta caaacatgtg ttagatgcta cagagctacc 1140

ggggacaatt ctgctacagt ggtgtctggg acagttgtaa atggtaatgg ggagcagggg 1200
gttgatgttc tggtccctac atctcaagta aagactagct gctgtccttc agggcaagat 1260
cgttgctcgc caatgcaccc tgcaagcaac gacgtgctga cagcactatt gctggcatta 1320
cctcaacata catggtctca aataaaagat acaatggtct tgcaggaaat agagaacctt 1380
gtctcagcag agaacctgcc tcctttgctg caagaagaga ttttgcacct gcgagggcag 1440
ttcctcctcc tcaagaaatg caaggataac aaggtagaag aagatttagc tgcacctccc 1500
ttctag

<210> SEQ ID NO 2
<211> 22
<212> DNA
<213> Artificial

<220>
<223>   FW1 primer

<400>
atggcgatgg cgggtttata tc

<210> SEQ ID NO 3
<211> 27
<212> DNA
<213> Artificial

<220>
<223>   FW2 primer

<400>
ggatgccatg aacacgactg atgaagc

<210> SEQ ID NO 4
<211> 22
<212> DNA
<213> Artificial

<220>
<223> RV1 primer
<213> Artificial

<400>
ctagaaggga ggtgcagcta aa

<210> SEQ ID NO 5
<211> 22
<212> DNA
<213> Artificial

<220>
<223> RV2 primer

<400>
caccctccta gttctttatt cc

<210> SEQ ID NO 6
<211> 22
<212> DNA
<213> Artificial

<220>
<223> RV3 primer

<400>
tttgcgactt tgaccgtgta tc

## Description

### FIELD OF THE INVENTION

The field of the present invention is related to the molecular biology area, particularly in the implication of plants' genes in sodium detoxification processes, the use of transformed organisms expressing these genes in constitutive or induced form, and bio-remedy methods to recuperate the medium. Thus, the present invention refers to the conclusion of a tool and a specific method to improve salinity tolerance of living organisms eliminating sodium (Na+) from waters, soils, mud and any other mediums containing this element, based on the use of phytochelatine synthase gene from *Nicotiana glauca.*

### BACKGROUND OF THE INVENTION

Soil salination is one of the most important concerns regarding agricultural development.

Global climatic changes produce an increase in hydric stress, a factor associated to saline stress, since hydric deficit is also a cause of salt concentration increase in soils.

It is known that salt concentration within the cell of many organisms fluctuates around 50-10mM, thus benefiting the proteic structure, for example, due to electrostatic forces. However, a 300-500mM concentration inhibits metabolic reactions, altering balance between electrostatic and hydrophobic forces (Serrano R (1996) Int Rev of Cyt 165:1-52.).

Cells response to sodium chloride (NaCl) is determined by a group of diverse mechanisms:
Na+ may enter the cell through various types of channels, "*voltage-dependent cation channels*" and "*voltage-independent cation channels*" (VIC). The VICs are the principal path to Na+ entrance to plant cells (Xiong L. et al. (2002) in Salt Tolerance. The Arabidopsis Book (American Society of Plant Biologists, pp 1-22*).* Due to the similarity between Na+ and K+, voltage dependent K+ carriers might facilitate the entrance of Na+. For example, Arabidopsis AtKHT1 (sodium carrier with sequence homology to HKT family of potassium carriers), is involved in the conduction of Na+ from stems to roots. This circulation seems to play an important role in plants' tolerance to saline stress (Berthomieu P et al. (2003) EMBO journal 22:2004-2014).

Variation in membrane potential (values ≤ 130 mV) enables the entrance of Na+. Hyper-polarization in the plasmatic membrane of yeast is produced by the absence of the *PMP3 (SNA1)* gene. Homologues in *A thaliana* are *RCI2A* and *RCI2B* (*BLT101* in wheat). The over-expression of *RCI2A* might ease the growth suppression and photo-oxidant damages reducing the entrance of Na+ in the roots (Mitsuya S. et al. (2006) Physiologia Plantarum 128:95-102).

The role played by Ca²⁺ in the intricate group of responses to NaCl has been recently clarified in various aspects. The over-expression of ACA4 (Ca²⁺ vacuolar ATPase of *Arabidopsis thaliana*) in yeast increases tolerance to salt (Geisler M. et al. (2000) Plant Ph ysiol 124:1814-1827).

Na+ is evacuated out of the cells by means of Na+/H+ anti-carriers located in the plasmatic membrane. Accordingly, the over-expression of the *SOS1* gene of *A. thaliana*, encoding for the anti-carrier of Na+/H+ of SOS1 plasmatic membrane, improves the tolerance to salinity (Shi H. et al. (2003) Nat biotechnol 21:81-85). Besides the extrusion of Na+ ions, the compartment effect in the vacuole is one of the most important causes of tolerance to salinity. The protons gradient enabling the anti-carrying is produced by H+-ATPases and H+-vacuolar pyrophosphatases (PPases). Transgenic plants over-expressing AVP1, vacuolar H+-PPase, show a saline tolerance correlated to increase in ionic content inside the plants (Yamaguchi T.et al. (2005) Trends Plant Sci 10:615-620). Likewise, plants over-expressing AtNHK1 (Na+/H+ vacuolar anti-carrier of *Arabidopsis thaliana*), were capable of growing, blossoming and producing seeds in presence of 200mM NaCl in transgenic plants of *Brassica napus* (Zhang H-X. Et al. (2001) Proc Natl Acad Sci USA 98:12832-12836) and transgenic tomato (Zhang H-X. et al. (2001) Nat biotechnol 19:765-768). The over-expression of AgNHX1 (proceeding from the halophyte plant *Atriplex gmelini*), BnNHX1 (*Brassica napus*), HbNHX1 (*Hordeum brevisubculatum)* and GhNHX1 (*Gossipyum hirsutum*) also play the same role (Yamaguchi T. et al. (2005) Trends Plant Sci 10:615-620). The heterologous expression of TsVP (H+-PPase cloned from *Thellungiella halophila*) in *ena1* mutant yeast (pump eliminating Na+ out of the cell) suppresses hypersensitivity to Na+. The tobacco transgenic plant over-expressing TsVP attains 60% more of dry weight than the wild type when exposed to 300mN NaCl (Gao F. et al. (2006) J Exp Bot 57:3259-3270).

Osmolytes also play a relevant role in tolerance to salinity. They protect against water loss and changes in the plasmatic membrane structure as a result of the elimination of ROS ("Reactive Oxygen Species") toxic effects generated by saline stress. Proline, glycine, bethaine, trehalose, manitol and sorbitol, abundantly produced and accumulated in cells treated with salt, represent an important component in responses to saline stress (Sahia C. et al. (2006) Physiol Plant 127:1-9). Over-expression of enzymes involved in the detoxification path of ROS (SOD, CAT, GST, APX, GPX) result in an increase in tolerance to saline stress (*Xiong L, Zhu J-K (2002)*). Transgenic tobacco seeds over-expressing a cDNA encoding for an enzyme with glutation S-transferase activity (GST) and glutation peroxidase (GPX), grow faster than control seeds when exposed to low temperatures and saline stress (Roxas V-P, Smith R-K, Jr, Allen E-R, Allen R-D (1997) Nat biotechnol 15:988-991). Glyoxalase I (gly I) and glyoxalase II (gly II) enzymes are necessary for detoxification form methylglyoxal and confer tolerance to salinity in tobacco transgenic plants (Singla-Pareek S-L, Reddy M-K, Sopory S-K (2003) Proc Natl Acad Sci USA 100:14672-14677).

There is a clear connection between oxidizing and osmotic stress through the so called "*Mitogen-Activated Protein Kinase*" (MAPK). The *EhHOG* gene, encoding an MAPK having an essential role in the path of yeast and other eukaryote osmo-regulation, was isolated from *Eurotium herbariorum* of the Dead Sea. When EhHOG was over-expressed in the *hog1* mutant of *S*. *cerervisiae,* the growth and aberrant morphology of *hog1* were restored in high osmotic stress conditions *(*Jin Y, Weining S, Nevo E (2005) Proc Natl Acad Sci USA 102:18992-18997).

Various genes induced by saline stress belong to the LEA family. Diverse types of Arabidopsis LEA are known; RD ("*Responsive to Dehydration*"), COR ("COId-*Regulated*"), LTI ("*Low Temperature-Induced*"), KIN ("*cold induced*"). All these types are induced by saline and hydric stress, low temperatures and ABA. Transgenic rice over-expressing SNAC1 ("*STRESS-RESPONSIVE NAC 1*") is more sensitive to ABA and looses water slower due to closure of the stomas; SNAC1 might improve tolerance to drought and to salinity in rice. (Hu H. et al. (2006) Proc Natl Acad Sci USA 103:12987-12992).

Thus, sensibility of plants to sodium presence is not a feature inherent to them, since many adaptations are known in soil as well as in seawater, at high saline concentrations, as is the case of halophyte plants. This indicates that tolerance might be resolved through gene transference. To date, genes described in defense against saline stress were involved in transport, extrusion, osmo-protection, vacuolar anti-carriers, transcription factors, etc.

Plants containing diverse groups of molecules used to ease effects of salinity have been developed in the last decades. For example, osmo-protectants, which are solutes compatible with proline (amino acids), glycine-bethaine, dehydrine and sugars (manitol, trehalose, etc) that work as osmolytes and protect cells from dehydration and thus loss of bulge, improve maintenance of roots and trigger in response to water deficit.

In August 1999 Eduardo Blumwald, an Argentinean scientist working in Toronto, publishes the use of a vacuolar anti-carrier of *Arabidopsis thaliana* that ejects H+ to cytoplasm while accepting Na+ ions (patent no. Ca 2,323,756 and afterwards patent US 6,936,750). This enables plants over-expressing it to be able to live in highly saline environments.

In August 2001 the same researcher, now in Davis, University of California, brings to light the obtention of a tomato plant genetically modified growing and developing in salty water irrigation. It is important to outline that even though all along the past century a good number of researchers have been trying to develop crop varieties tolerant to salt using classical improvement techniques, none of the efforts rendered the expected results.

In 2001 the demonstration that atHKT1 is a Na+ carrier to the interior of the *Arabidopsis thaliana* root (Rus et al., Plant Physiology 136:2500-2511 (2001*)* is made public. Thus, theoretically, the over-expression of this gene in a plant would allow a greater income of Na+ through the roots of individuals modified with said gene.

The Spanish Patent Application No. 2,173,019, published in 2002, defines the use of the sodium ATPasa gene of *Neurospora crassa* in the improvement of tolerance to salinity.

Now, the authors of the present invention have developed a method to improve the tolerance to salinity based, for the first time, on the use of molecules capable of linking directly to sodium in order to block its toxic action inside the cell.

These molecules are the phytochelatines (PCs). PCs are peptides rich in cysteine that are not genetically codified. Its synthesis starts or is induced due to the presence of heavy metals, as cadmium, with the concourse of the phytochelatine synthase enzyme (PCS) using GSH as substrate to form the peptide [γ-Glu-Cys]n=2-11-Gly (PCs) (Steffens, J.C. (1990) The heavy metal-binding peptides of plants. Ann. Rev. Plant Physiol. Plant Mol. Biol. 41,533-575).

The PCS gene of different species has been used to improve tolerance and accumulation of heavy metals in diverse vegetable species as a solution to the problem of soils contamination by these contaminants, that is, selected plants have been endowed with a higher capacity to tolerate and, what is more interesting, accumulate heavy metals through phyto-extraction. Thus the PCs seem to play an essential role in the regulation of cellular equilibrium in ions of "free" and complexed heavy metals through a simple and efficient mechanism (Erwin G. et al. (1989) Proc Natl Acad Sci USA 86:6838-6842. Moreover, many authors previously suggested that PCs might play a role in detoxification of heavy metals (Cobbett C (2002) Annu Rev Plant Biol 53: 159-182). This has resulted in patents of genes encoding for phytochelatines synthases (US 6,489,537 and US 6,844,485), but in no case have they been used to stand salinity, so the proposal herewith presented generates a new method to confront contamination by salts or salination.

On the other hand, the state of the art accounts for non biological methods used for the restoration of saline-sodic soils that generally consist in the addition of calcium sulfate (gypsum) to facilitate the cationic exchange and wait for diverse rainfall washes of the substituted cations. However, the migration of salts to deeper horizons is not the solution to the problem since it could ascend again by capillarity or contaminate water reservoirs.

To this end, the present invention also provides a procedure to reduce or eliminate sodium from any medium (solid, liquid or gaseous) containing said alkaline metal, consisting in the use of PCs in a living organism whose capacity to resist the effects of salinity is limited and subtract it from a concrete means through its accumulation in it.

Besides the use in this method of already known sequences encoding for PCSs of different species, the authors of the present invention have sequenced for the first time the gene of *Nicotiana glauca* PCS, which can be expressed in constitutive or induced form in *N. glauca* or in any other living organism with limited tolerance to salinity.

The method object of the present invention presents significant advantages with respect to procedures developed in the state of the art to stand salinity. On one hand, it is a method of easy application and great usefulness, since it enables the direct application to the contaminated medium, which means, in the case of salinized soils, avoiding the loss of soil due to the superficial wash of sluice waters from those soils that do not allow the growth of wild plants. Besides, it presents economic benefits since it takes advantage of the capacity of living organisms to decrease erosion and/or accumulate the salts.

### BRIEF DESCRIPTION OF THE DRAWINGS

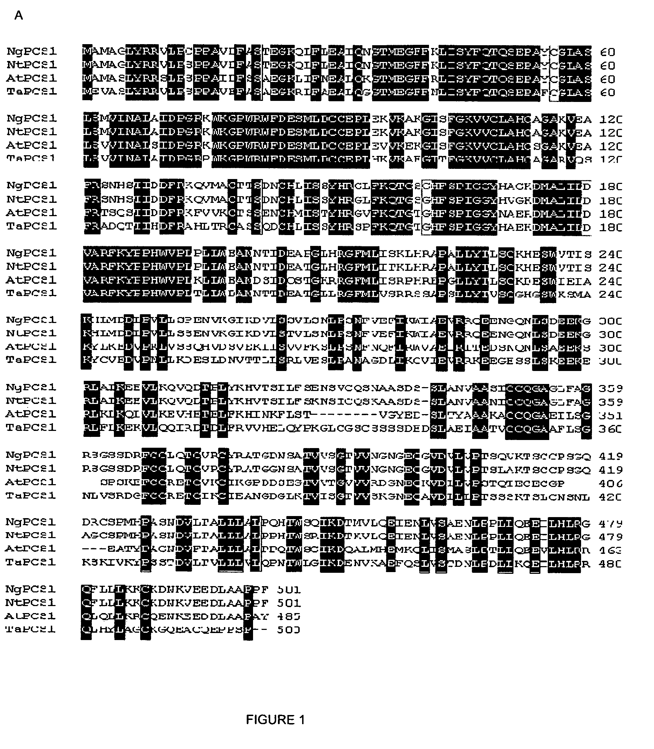

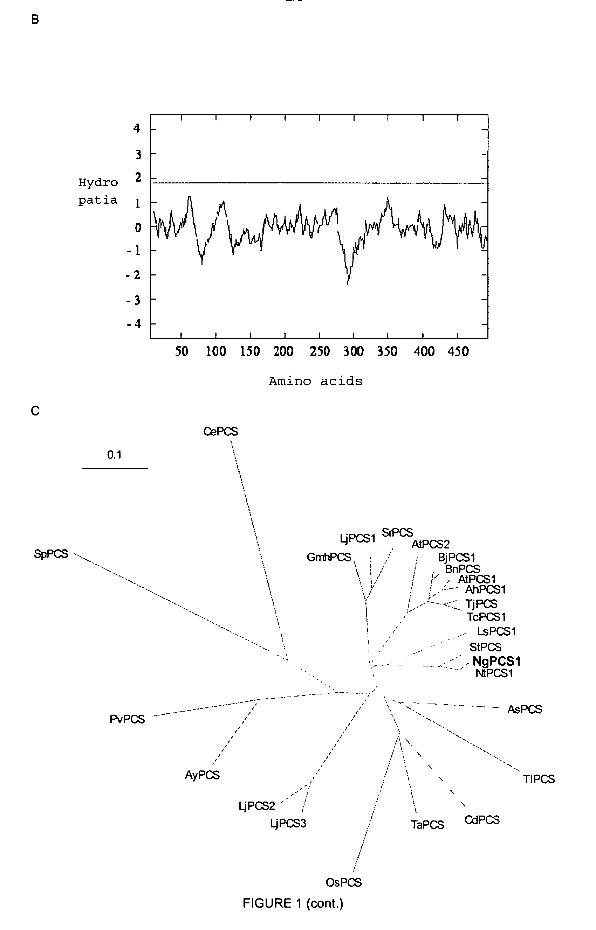

**Figure1****. Comparison between known NgPCS1 and PCSs. A. CLUSTAL W Alignments of the amino acids sequence of PCS1 of Nicotina glauca (NgPCS1), N. Tabacum (NtPCS1), A. thaliana (AtPCS1) and T. aestivum (taPCS1).** Identical amino acids are in black. In gray: catalytic triad formed by Cys56, Hys162 and Asp180 similar to papain (papain-like) indispensable for the catalysis. **B. Hydropathy profile of Kyte-doolittle of NgPCS1.** Values nearing 2 indicate those enzyme zones that are probably insert in the membrane. **C. Phylogenetic analysis of PCSs of different species.** Tree without root constructed with the neighbour-joining method (Clustal W). The bars represent the genetic distance (0,1 substitutions by site). The encoding sequences of PCS genes are detailed as follows, the species and access number are in parenthesis:
   *AhPCS1* (*Arabidopsis halleri,* AAS45236.1), *AtPCS1* (*Arabidopsis thaliana,* AAD16046.1), *AtPCS2* (*Arabidopsis thaliana*, AAK94671.1), *AsPCS* (*Allium sativum,* AA013809.1), *AyPCS* (*Athyrium yokoscense*, BAB64932.1), *BjPCS1* (*Brassica juncea,* BAB85602.1), *BnPCS* (*Brassica napus*, CAK24968.1), *CdPCS* (*Cyonodon dactylon,* AAO13810.2), *CePCS* (*Caenorhabditis elegans,* NP_4964575.3), *GmhPCS* (homo-phytochelatine synthase, *Glycin max*, AAL78384.1), *NgPCS1* (*Nicotiana glauca*), *NtPCS1* (*Nicotiana tabacum,* AAO74500.1), *LsPCS1* (*Lactuca sativa*, AAU93349.1), *LjPCS1* (*Lotus japonicus,* AAQ01752.1), *LjPCS2* (*Lotus japonicus* AAT80341.1), *LjPCS3* (*Lotus japonicus,* AAY81940), *OsPCS* (*Oriza sativa*, AAO13349.2), *PvPCS* (*Pteris vittata*, AAT11885.1), *SpPCS* (*Schizosaccharomyces pombe*, Q10075), *SrPCS* (*Sesbania rostrata*, AAY83876.1), *StPCS* (*Solanum tuberosum*, CAD68109.1), *TcPCS1* (*Thlaspi caerulescens,* AAT07467.1), *TjPCS* (*Thlaspi japonicum*, BAB93119.1), *TaPCS* (*Triticum aestivum*, AAD50592.1) and *TIPCS* (*Thypha latifolia*, AAG22095.3).

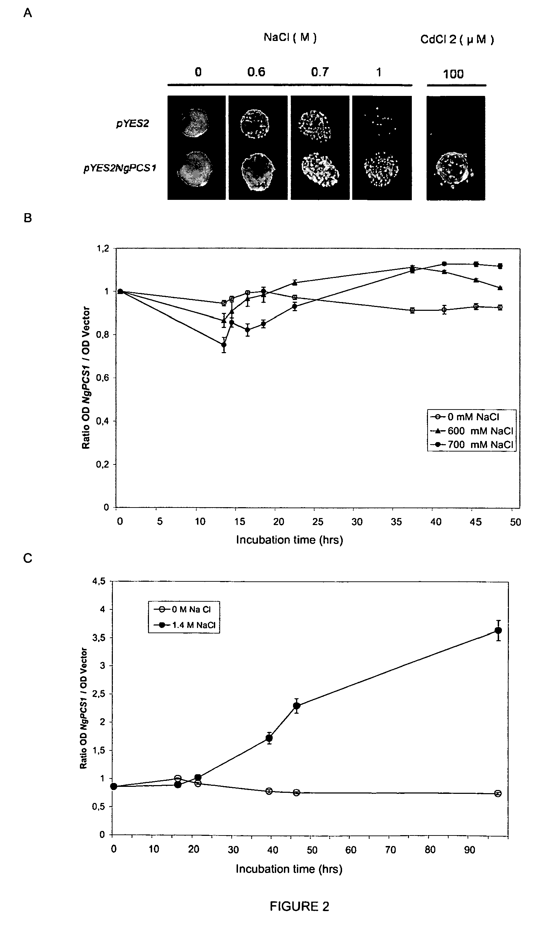

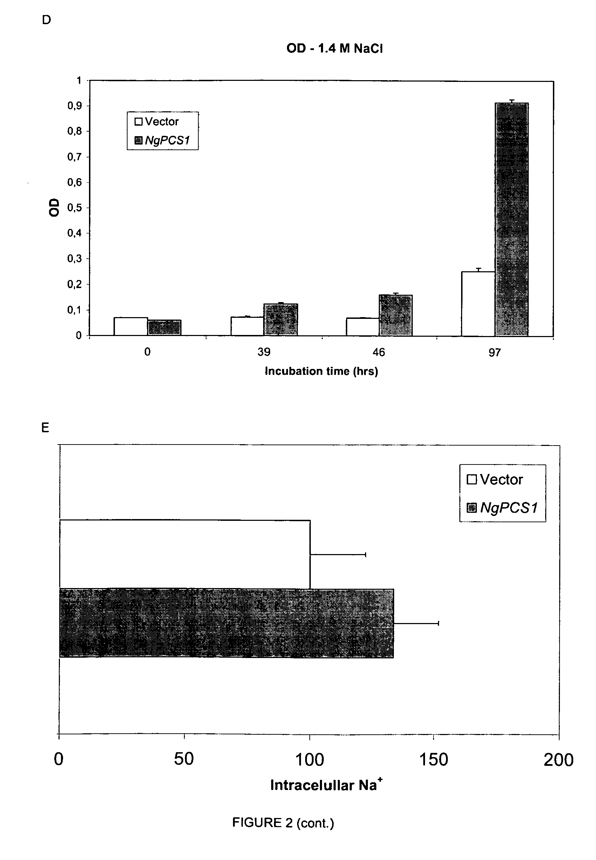

**Figure 2****. Comparative growth in Cd²⁺ and NaCl. A. Expression of *NgPCS1* in yeast in a medium containing Cd²⁺ and Na⁺.** Dripping test. Cells (DO at 600 nm of approximately 1,4) in stationary growth step were deposited in serial dilutions in a SD minimum medium (1% sucrose/1% galactose) supplemented with appropriate amino acids and NaCl 0, 0,6, 0,7 1 M and CdCl₂ 100µM respectively. The figures show the growth of dilutions 1:20 after 3 days (4 days for the 1 M concentration). **B. Growth of yeast cells expressing *NgPCS1* in a medium with NaCl 0,6 and 0.7 M.** The figure shows the ratio comparison of growth vector p*YES2NgPCS1*/ vector p*YES2* empty in cells without NaCl and in cells with NaCl against incubation time (in hours). The cells were grown at a DO of 1 at 600 nm, and inoculated with a 10⁶ cells concentration by ml in a liquid medium without NaCl or with NaCl 0,6 M, 0,7 M, and 1,4 M. **C. Comparison of growth ratios of vector p*YES2NgPCS1*/vector pYES2 empty in cells without NaCl and with NaCl 1,4 M.** Ratios have been used to minimize those growth differences not owing to types of stress specifically studied since the sole presence of vector p*YES2NgPCS1* in yeasts produces differences. **D. Comparison of optic density between cells without NaCl and with NaCl 1,4 M.** Growth model in minimum medium represented by DO against time in hours measured during a 4 days growth period. The differences in absolute growth value are observed for the 4 selected times. **E. Relative intracellular Na+ concentration content.** The comparison of the intracellular concentration of Na+ in cells containing the vector pYES2 empty and cells containing p*YES2*/*NgPCS1.* The yeast cells were grown at a DO of 0,6 at 600 nm. NaCl was added at a final concentration of 1,4 M. The values in cells with p*YES2* empty were taken as 100%.

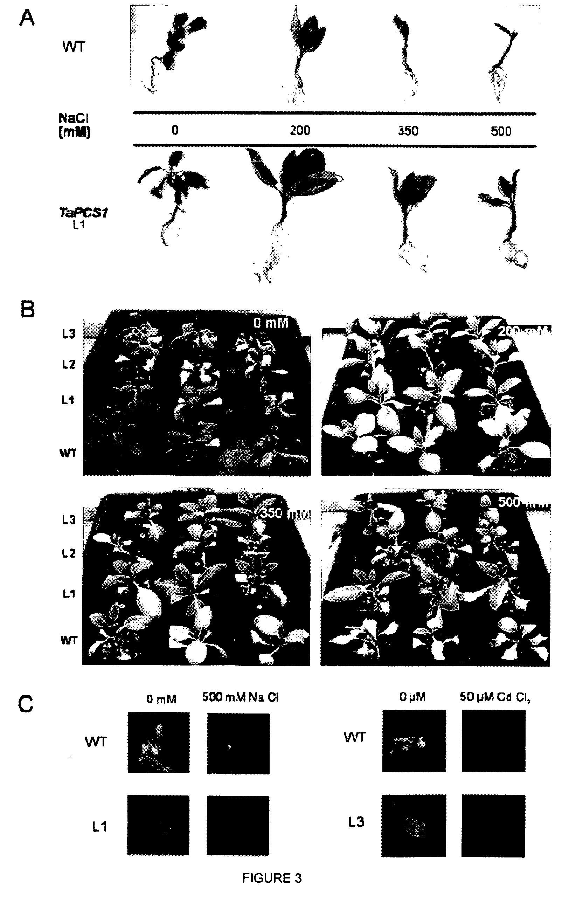

**Figure 3****. Expression of *TaPCS1* in hydroponic media and soils. A. Plant growth in hydroponic conditions.** WT: wild phenotype (wild type), *TaPCS1*: plants that over-express *TaPCS1.* **B. Growth in pots**. L1, L1and L3, are three different lines containing over-expressed *TaPCS1.* **C. Confocal microscopy of radicular tissues.** DHE was used as informer of oxidant stress. Control: without NaCl. The modified lines L1 and L3 were selected as representatives of 4 repetitions.

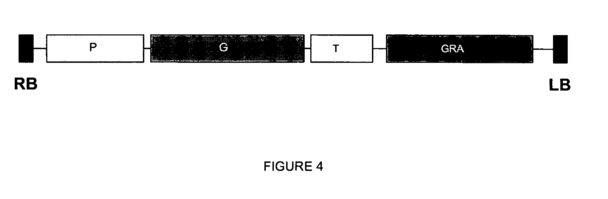

**Figure 4****: ADN transference scheme (tDNA). LB:** left termini, **LR**: right termini, **P**: promoter, **G**: gene of interest; **T:** terminator; **GRA:** gene resistant to antibiotics.

### OBJECT OF THE INVENTION

The object of the invention is the *NgPCS1* gene encoding the phytochelatine synthase of *Nicotiana glauca* with the SEQ ID NO 1 sequence.

It is also object of the invention the use of the *NgPCS1* gene in a method to improve the tolerance to salinity and accumulation of sodium in any living organism.

Another object of the invention is the use of the *NgPCS1* gene in a method to recuperate a salinized medium through modified organisms expressing a phytochelatine synthase codified by SEQ ID NO 1 or sequences having at least a 35% similarity with SEQ ID No. 1.

Finally, it is an object of the invention the use of phytochelatines obtained *in vivo or in Vitro* through enzymatic reaction mediated by phytochelatine synthase codified by the SEQ ID NO 1, or sequences with at least a 35% similarity, as sodium chelators.

### DESCRIPTION OF THE INVENTION

In a main aspect, the invention refers to an isolated sequence of a nucleic acid encoding for phytochelatine synthase of *Nicotiana glauca* (NgPCS1), characterized by the SEQ ID NO 1.

The term "encoding" refers to a property inherent of specific nucleotide sequences in a polynucleotide such as gene, cDNA or mRNA serving as mould for the synthesis of polymers and macromolecules in biological processes or in processes carried out *in vitro.*

Another main embodiment of the invention contemplates a vector comprising the SEQ ID NO 1. In a preferred form, said vector is a plasmid.

Another main embodiment of the invention, refers to a stable transgenic organism (cell or genetically modified organism) comprising the sequence SEQ ID NO 1.

Phytochelatine synthase of *Nicotiana glauca* (NgPCS1) located in the cytoplasm, is the enzyme that mediates the production of phytochelatines (PCs), using glutathione (GSH) as substrate. The over-expression of *NgPCS1* results in an increased tolerance to Na+, as well as tolerance to heavy metals already observed in other PCSs. The mechanism through which an improvement of tolerance is produced is the chelation, through the PCs, of Na+ ions and the further seclusion in vacuoles of the PC-Na+ complexes.

Thus, another main aspect of the invention relates to the use of the SEQ ID No 1 sequence to improve the tolerance to salinity and/or accumulation of sodium (Na+) in a living organism (animal, vegetal or microorganism).

This has enabled the authors of the present invention to develop a method to improve the tolerance to salinity and/or accumulation of Na+ in a living organism comprising the following steps:
- transforming the living organism with the SEQ ID NO 1, or a sequence with at least a 35% similarity with the SEQ ID NO 1; and
- expressing the sequence (SEQ ID NO 1, or a sequence with at least a 35% similarity with the SEQ ID NO 1), controlled by functional regulatory sequences in the living organism.

Sequences with at least a 35% similarity with the SEQ ID NO 1 encompass those genes having phytochelatine synthase function, that is, from eukaryotes as the *Caenorhabditis elegans* worm and bacteria to plants (having a higher similarity).

The transformation step is carried out by any of the known state of the art methods. In a particular embodiment, in a first step the construction of a vector comprising the SEQ ID NO 1, or a sequence with at least a 35% similarity with the SEQ ID NO 1 is carried out and, afterwards, said vector is introduced in the living organism.

To improve tolerance to sodium in a living organism it must express the gene of the sequence introduced under the transcriptional control of a regulatory sequence that may be constitutive (always facilitating the expression) or induced (facilitating the expression only if there is sodium).

In the present invention, the term "functional" refers to the regulatory sequences having effect on the functionality of the gene as to the transcription (start and ending) and translation (start and ending) of messenger RNA and others not described.

Among the regulatory sequences of the present invention are the promoters and others less common as certain introns, the sequences of transcription terminus and sequences of start and ending for the posterior translation of messenger RNA. Phytochelatine synthase of the species *Nicotiana glauca* may be thus expressed in constitutive or induced form in *N. glauca* or in any other living organism whose capacity to stand the effects of salinity is limited.

The constitutive expression of PCs produces an improvement in the growth of yeasts and plants so as to be used to solve various problems: (a) cultivation of numerous plants in salinized soils or in waters with saline contamination, that might generate two direct benefits, the revaluation of abandoned lands on account of salination thanks to biomass production, as well as the restoration of the same to be cultivated again and (b) the cultivation of modified microorganisms in saline contamination media to reduce the content of salts in such media.

In a particular embodiment, the living organism used in the method of the present invention is a yeast, preferrably *Saccharomyces cerevisiae.*

In another particular embodiment, the living organism is a plant, preferably *Nicotiana glauca.*

In another main aspect of the invention, the use of the SEQ ID No 1 sequence is used to reduce or eliminate sodium from a liquid, solid or gaseous medium.

This application enables the development of a method to reduce or eliminate Na+ from a liquid, solid or gaseous medium based on the following steps:
i. transforming living organisms with the SEQ ID NO 1 or a sequence with at least a 35% similarity with the SEQ ID NO 1;
ii. identifying transformed organisms in i) through selection with antibiotic;
iii. seeding the salinized medium with the organisms identified in ii);
iv. cultivating the organisms during an appropiate lenght of time, and
v. collecting the organisms

In a preferred form, the genetic transformation process is carried out through electroporation. This process succeeds in the production of host cells of *Escherichia coli* or *Agrobacterium tumefaciens* carrying a vector with the desired insert (alter being selected with the corresponding antibiotic). As previously clarified, these cells are also considered in the present invention as transgenic organisms, though they are not directly employed in the sodium link in a salinized medium but serve as amplifier means in the case of *E. coli* and as instrument of infection in the case of *A. tumefaciens.*

To carry out the sodium elimination process it is crucial to be sure that the organisms used contain the transgene inserted in its genome or otherwise express it through vectors. Thus, to obtain organisms expressing the sequence SEQ ID NO 1, or a similar sequence in at least a 35%, it is necessary to carry out a selection step. The selection is carried out with antibiotics, since the transgenic organism incorporates through the vector a gene resistant to antibiotics (GRA). It is located next to the gene of SEQ ID NO 1, between the left border (LB) and the right border (RB) defining the flanking regions of the transference DNA (tDNA) (figure 4).

In case microorganisms expressing the gene to eliminate the salts from a salinized medium are used, the collection of organisms shall be carried out through floculation, precipitacion, centrifugation or any other method enabling the separation of microorganisms that have accumulated the salts of the medium.

In case plants expressing the gene to eliminate the salts from a salinized medium are used, the plant is to be collected, triturated before or after being dried and the remains are to be dumped in a residues' dumping place according to the legislation.

Finally another main embodiment of the invention comprises the use of phytochelatines (PCs), obtained *in vivo or in vitro* by enzymatic reaction mediated by phytochelatine synthase codified by the SEQ ID NO 1, or sequences with at least a 35% similarity, as sodium chelants.

### Examples

### Example 1. Phytochelatine synthase from N. glauca is very similar to the N. tabacum homologous.

The design of primers in conserved zones of the encoding region of the PCS gene of *N. tabacum* led to the amplification of a PCR fragment of an expected size (1,5 Kb). The open reading frame codified a protein (NgPCS1) with a molecular mass of 55,14 kD; 501 residues of amino acids and a pH of 6,32. The hydropathy profile was correlated to a cytoplasmatic protein (**figure 1B**). The new protein was compared to the PCS of *A. thaliana* (access numer AAD16046.1), *N. tabacum* (access number AY235426) and *T. aestivum* (access number AAD50592.1), resulting in the following identity percentage: 96% in relation to NtPCS1, 64% in comparison to AtPCS1 and 59% to TaPCS1. This high identity among the sequences of PCSs in plants belonging to different families such as *Brassicaceae, Poaceae* y *Solanaceae*, indicated an elevated conservation and, thus, an important role of these proteins in the vegetal kingdom. The identity percentage between the sequences of proteins of the most investigated PCS of *T. aestivum* (TaPCS1) and the PCS of *A. thaliana* (AtPCS1) was 58%, similar to the percentage found for the new notified PCS of *N. glauca* and the PCS of *T. aestivum* (59%). Cohesively, Cys⁵⁶, Hys¹⁶² y Asp¹⁸⁰ described as a catalytic triad similar to papain (papain like) indispensable for the (Rea P-A (2006) Proc Natl Acad Sci USA 103:507-508) catalysis, were also conserved in NgPCS1 (**figure 1A**). A rootless tree with 24 sequences of PCSs related to the foreseen primary structure of NgPCS1 (Blastp of the NCBI database) was obtained (**figure 1C**). The following were identified among the separated clusters: the family of the *Brassicaceae* cluster formed by the *Arabidopsis*, *Brassica y Thlaspi* genus, and the *Nicotiana* and *Solanum* belonging to the *Solanaceae* cluster family.

### Example 2. Over expression of NgPCS1 in yeasts leads to a tolerance to Cd²⁺ and an accumulation and tolerance to Na⁺.

Cloning and research of different PCSs led to the conclusion that these genes can confer accumulation and tolerance to Cd²⁺ (Clemens S. et al. (1999) EMBO J 18: 3325-3333*. 27*). As expected, *NgPCS1* can also confer tolerance to Cd²⁺. This is especially evident in this work from the experiments carried out at a 100 µM concentration (**figure 2A**). This fact totally agrees with former results obtained wherein the over expression of *TaPCS1* was generated in yeasts in similar Cd²⁺ concentration conditions and using the same vector (*Clemens S. et al. (1999)*), thus establishing an homologous function of both genes *TaPCS1* and *NgPCS1* as well as a structural similarity.

*NgPCS1* conferred tolerance to Na+ when it over-expressed in yeasts in concentrations of NaCl oscilating from 0,6 to 1 M (**Figure 2A**), indicating a relevant function for this type of enzyme in tolerance to saline stress beyond the well known role performed in the accumulation of heavy metals. The growth mediated by *NgPCS1* with saline stress treatment analyzing the kinetics of growth proportions at concentrations of NaCl of 0,6 and 0,7 M was also investigated (**figure 2B**). At both concentrations, the cells containing p*YES2NgPCS1* exhibited a clear initial growth disadvantage in comparison with the empty vector *pYES2.* However, after 22 hours of treatment, the cells exposed to a 0,6 concentration M first and 0,7 M afterwards, exhibited a better growth than control cells. Finally, the 0,7 M concentration yielded a greater growth difference than 0,6 M and thus, higher NaCl concentrations yielded a superior improvement of the mediated growth by *NgPCS1.* Consequently, with 1,4 M NaCl the growth of yeast cells through the presence of NgPCS1 in said cells was improved (**Figure 2C**). As observed at 0,6 and 0,7 M concentrations, though there was no perceptible difference in growth proportion during the first 22 hours, the effect generated by p*YES2NgPCS1* increased in time, leading to a greater difference at the end of the measurements, confirming a late response mediated by *NgPCS1* in tolerance to Na+. Experimental growth conditions carried out in this work, favouring the slow growth (minimum synthetic medium, without free glucose), enabled a better evaluation of the factors stimulating the growth in saline stress conditions. There was an increase in the difference of DO600 in favour of the cells containing p*YES2NgPCS1,* clearly appreciated after 39 hours of treatment and increasing spectacularly at 97 hours (**figure 2D**) indicating that NGPCS1 improved drastically the tolerance to salinity. However, the toxicity of NaCl can be divided in two components: hydric stress and damage by Na+. Thus, the following question came forth: NgPCS1 improves the tolerance to NaCl, for example, increasing only the retention of water, but not the accumulation of NaCl? Or it improves the tolerance and the accumulation? To respond this question, the accumulation of Na+ in the cytoplasm of yeast cells with or without *NgPCS1* was analyzed, demonstrating that NgPCS1 leads to a greater accumulation of Na+ inside yeast cells containing the vector *pYES2NgPCS1* (**figure 2E**).

### Example 3. Phytochelatines confer tolerance to Na+ in plants and diminish oxidating stress.

To examine the capacity of PCS to improve the tolerance to Na+ in plants, *N. glauca* specimens over expressing wheat PCS (*TaPCS1*) previously tested to determine the accumulation of heavy metals, were grown. Gisbert C. et al. (2003) Biochem Biophys Res Commun 303:440-445) (Martínez M. et al. J (2006) Chemosphere 64:478-48524).

The binary vector pBI121 (Clontech) was used for the transformation. The GUS gene of the binary vector was substituded by the encoding DNA of wheat phytochelatine synthase *TaPCS1* through the *Bam*HI and ECL136II restriction sites. Next, the introduction of the obtained plasmid, containing the *TaPCS1* cDNA in *Agrobacterium tumefaciens* C58C1RifR was carried out, and afterwards the transference by infection of *N. glauca* plants was carried out. The new construction was electropored in *Agrobacterium tumefaciens* cells. The transformants were selected in LB plates with kanamicine and contacted with 1 cm diameter disks during 10 minutes with a suspension of *A*. *tumefaciens* containing the desired construction. After generating adult plants through the regenerating program *in vitro* of *N*. *glauca* explants resistant to kanamicine, the seeds of the different transgenic lines obtained were recollected, selecting those containing one or various integrations in the plant genome, recovering those capable of growing in presence of the antibiotic kanamicine. The last step consisted in verifying if the integration of *TaPCS1* cDNA to the *Nicotiana glauca* genome sensibly improved the tolerance of the plant to Na+. The tolerance to Na+ study was carried out germinating the transgenic seeds in a substrate with vermiculite and dolomite as well as in hydroponic conditions, applying NaCl at 7 and 2 weeks respectively, to a final concentration of 200, 300 and 500 mM.

Two aspects were evident. In the first place, both types of specimens, the wild type and those that over expressed PCS, followed exactly the same growth pattern in relation to the tested NaCl concentrations, indicating a net effect of PCS in the tolerance to NaCl (**figure 3A**). In second place, the specimens of *N*. *glauca* needed NaCl to improve growth, at least in the hydroponic conditions tested in this work. Both types of plants followed the same pattern but the OMGs (Genetically Modified Organism) exhibited a better growth in all cases. This similarity in growth patterns only differed in an improvement factor, indicating that the over-expression with NaCl of *TaPCS1* in *N*. *glauca* is transparent in the sense that the gene does not change the inner balance inside the cell and only improves the quantity of metabolized salt. When halotolerance in solid media was analyzed, the plants also exhibited the typical pattern observed in hidroponic conditions (**figure 3B**). As determined in hidroponic crops, the presence of NaCl improved the growth and confirmed that 200 mM is the optimum NaCl concentration for all the plants used in the experiment, even better that the groth obtained in the absence of NaCl. However, the growth at 350 mM and 500 mM was anomalously high considering the magnitude of the foliar surface. In relation to the role played by the PCS, the observed tolerance in NaCl 500 mM was especially evident since the specimens of wild type could not survive. Oxidating stress was lessened by the PCS (L1 and L3; (**figure 3C**). The PCS only increased quantitatively the natural trend of *N*. *glauca* to attain an improved growth with NaCl.

### Materials and methods used

### Cultures, transformation and yeasts growth assays

The strain of *Saccharomyces cerevisiae*, YPH499 (*MAT*a *ura3-52 leu2Δ1 lys2-801 Ade2-101 trp1Δ63, his3*-D*200* was used in this study. For growth assays in solid and liquid media, *S*. *cerevisiae* cells were grown in minimum synthetical medium (SD) with or without 2% agar bacto, respectively, containing 1% sucrose, 1% galactose, 0,7% nitrogenous base for yeasts without amino acids and with ammonic sulfate (Pronadisa) and MES-Tris 50 mM (pH 6,0). The SD medium was complemented with adenine (30µg/ml), histidine (30µg/ml), leucine (100µg/ml), lysine (100µg/ml) and tryptophan (80µg/ml). The transformation through the procedure with litium acetate and the selection of the transformants in yeasts was carried out as described (Ito H, Fukuda Y, Murata K, Kimura A (1983) J Bacteriol 153:163-168*)* and using the URA3 marker for the selection in yeasts. The yeasts cells carrying the empty vector p*YES2* were used as negative control. To investigate the kinetics of saline stress, cells were grown up to a DO at 600 nm of 1, approximately, and were inoculated at a concentration of 10⁶ célls per ml in liquid medium without NaCl or containing 0,6 M, 0,7 M and 1,4 M of NaCl. For dripping assays, cells were grown to saturation in SD diluted with water (1/2, 1/5, 1/10, 1/20, 1/100, 1/1.000 y 1/10.000), and distributed through replica plater 8 x 6 array (Sigma-Aldrich) in plates containing 0,6 M, 0,7 M y 1 M de NaCl y CdCl₂ 100 µM.

### Vegetable materials

For the planthouse experiment, seeds of *N. glauca* (wild type) and three F3 different transgenic lines (TaP12, TaP17 and TaP18, lines L1, L2 y L3 respectively) were sterilized as follows: the seeds were submerged in 30% commercial lye, plus 0,01% Triton X-100 detergent during 7 minutes to avoid fungal and bacterial growth, a second washing was carried out afterwards using a dissolution of 70% ethanol in water with 0,01% Triton X-100. Finally, the seeds were 5-folded consecutive washed with deionized water each lasting 5 minutes to eliminate any remainder of disinfectant dissolution. The submerged seeds were placed in Petric plates with a medium prepared with agar 6 g/liter, MS salts (Murashige T, Skoog F (1962) Physiol Plant 15:473-497) and sucrose 10 g/liter at pH 5,7 tamponed with MES ( 2-[N-morfoline acid] ethanosulphonic) 0,25 g/liter. At ten days (when the first leaves had developed), three plantules per line and pot treatment containing vermiculite and dolomite in the same proportions were transplanted and covered with film during some days to obtain better acclimate conditions. The six weeks plants were placed in a different tray for each treatment and were watered once a week with or without NaCl 200, 350 or 500 mM during two weeks. For the *in vitro* experiment, three plantules were placed for WT (wild type) and each line of *N. glauca,* growing in sterile conditions as described in (Gisbert C. et al. (2003) Biochem Biophys Res Commun 303:440-445), in 50 ml Falcon tubes containing MilliQ water with or without NaCl 200, 350 and 500 mM at room temperature in a soft shaker (25 rpm in an ELMI S4 shaker) during 7 days.

### NgPCS1 cloning

The encoding DNA was synthesized from 2 µg of total isolated RNA of *N. glauca* leaves through reverse transcriptase of M-MuLV (virus of Moloney murine leukemia) with an oligo(dT)₁₈ primer (kit synthesis Ferment of the first strand encoding DNA) according to recommended procedures in the kit. A microliter of produced encoding DNA was used as mould in a reaction of conventional PCR 50 µL. Design of the primers for polymerase chain reaction (PCR): the conserved domain in the N-terminal termini for *NtPCS1*, *AtPCS1*, *TaPCS1, BjPCS1* y *OsPCS1* was observed. Two different primers in the 5' termini, FW1 (SEQ ID NO 2) and FW2 (SEQ ID NO 3) were designed. The encoding sequences in the C-terminal region analyzed for this gene are less conserved than those of the N-terminal extreme. Thus, the sequence of *NtPCS1* RNA messenger was used, designing three different primers (SEQ ID NO 4), RV2 (SEQ ID NO 5) y RV3 (SEQ ID NO 6).

After carrying out the experiments of PCR using different combinations, only two bands with the expected size of 1,5 KB were obtained, corresponding in both cases to the primers FW1/RV1 and FW2/RV2. Using an agarose gel with 1% TAE buffer, the reactions of PCR were carried out and extracted through pressure-freezing of the cut band and DNA precipitation through 1/50 volumes of NaCl 5M and 2 volumes of absolute EtOH. After measuring the DNA concentration in a NanoDrop ND-100 spectrophotometer, the amplified fragments were cloned in the pGEM-T Easy vector (Promega, Southampton, UK).

*E. coli* DH5α was used as host. After selecting the right transformants and isolating the plasmidic DNA (Marligen Bioscience, quick plasmids' isolation system) the cloned fragments were sequenced in a DNA ABI Prism (Perkin-Elmer) sequencer using the sites T7 and SPC6 located in the vector. Both complete sequences of the 1,5 Kb fragments were aligned with *NtPCS1* using the William Pearson LALIGN program, observing a 93% identity in the sequence of the FW1/RV1 fragment. The sequence of the second fragment, FW2/RV2, revealed a 91% identity between the nucleotides 592 and 1501 of the *NtPCS1* encoding sequence. Alignment of both sequence fragments resulted in the same nucleotides composition between the 592 position and the terminal codon. The correct C-terminal sequence corresponding to the RV1 primer was assayed using the second *NgPCS1* sequenced fragment. *NgPCS1* was directionally subcloned in the *Kpn*I/*Bam*HI sites of the pYES2 expression vector through a new amplification by PCR with the primers FW2 and RV2 and with the additional *Kpn*I y *Bam*HI sequences, respectively. pYES2 includes the *Amp* of *E*. *coli* gene, the selectionable *URA3* yeasts marker and the inducible promoter GAL1 for expression in yeasts cells. *E*. *coli* was transformed by electroporation selecting the transformants for Amp^{r}. The plasmidic DNA of the correct clone containing *NgPCS1* in p*YES2* was transformed in the yeast strain YPH499 and was selected as previously described. The sequences of *NgPCS1*, *NtPCS1*, *AtPCS1* y *TaPCS1* were aligned with the CLUSTAL W (Thompson J-D, Higgins D-G, Gibson T-J (1994) Nucleic Acids Res 22: 4673-4680) program. A hydropathy profile of Kyte-Doolittle was built to learn the hydrophatic character of NgPCS1 (Kyte J, Doolittle R (1982) J Mol Biol 157:105-132).

### Intracelular Na+ concentrations measurement.

Yeast cells were grown in 20 ml of SD with the appropiate amino acids measuring the absorbance at 600 nm up to 0,6. Then, NaCl was added up to a final NaCl concentration of 1,4 M and incubated at 28°C shaking (150 rpm) during 3 hours, centrifugating during 5 minutes at 7,000 rpm (Beckman JA-20 rotor) and washed four times with 10 ml of a dissolution containing MgCl₂ 20 mM and sorbitol 1,5 M. Finally the intracellular content was extracted through incubation with 0,5 ml MgCl₂ 20 mM solution during 12 minutes at 95°C. After centrifugation during 2 minutes at maximum speed, the aliquots of the supernatant were analyzed with an atomic absorption spectrometer (Varian) in the flame emission mode.

### O₂⁻ Determination

The O₂⁻ superoxide anion was detected, as described by Yamamoto Y. et al. (2002) Plant Physiol 128:63-72*,* using dihydroetide (DHE), a reduced form of etide bromide that is not fluorescent and can passively pierce the membrane of living cells. Once in the cell, it oxidizes to yield a fluorescent colorant that links to nearby DNA. Production of O₂⁻ in the roots of *N*. *glauca* was observed after tinging the roots with DHE 10 µM in CaCl₂ 100 µM, at pH 4,75 during 13:30 h. Fluorescence images were obtained with a reverse Leica TCS SL confocal microscope. For the detection of the DHE, samples were excited at 488 nm using an argon laser and the emission was measured between 550 and 620 nm. The measurements by confocal microscopy and fluorescence were repeated at least five times with similar results. *N. glauca* seeds were grown durign six weeks in MS medium as described for vegetable materials. The DHE was administered after nine days of accomodation to hydroponic conditions.

## Claims

1. Isolated frequency of a nucleic acid encoding for phytochelatine synthase of *Nicotiana glauca* **characterized by** the SEQ ID NO 1.

2. Vector comprising the sequence of claim 1

3. Vector, according to claim 2, wherein said vector is a plasmid.

4. Genetically modified organism with the sequence of claim 1.

5. Use of the sequence of claim 1 to improve the tolerance to salinity and/or accumulation of sodium in living organisms.

6. Method to improve the tolerance to salinity and/or accumulation of sodium in a living organism comprising the following steps:
a. transforming the living organism with the SEQ ID NO 1 or a sequence with at least a 35% similarity with the SEQ ID NO 1; and
b. expressing the sequence controlled by functional regulatory sequences in the living organism.

7. Method, according to claim 6, wherein the living organism is a yeast.

8. Method according to claim 7, wherein the living organism is *Saccharomyces cerevisiae.*

9. Method, according to claim 6, wherein the living organism is a plant.

10. Method, according to claim 9, wherein the plant is *Nicotiana glauca.*

11. Use of the sequence of claim 1, to reduce or eliminate sodium from a liquid, solid or gaseous medium.

12. Method to reduce or eliminate sodium from a liquid, solid or gaseous medium based on the following steps:
a. transforming living organisms with the SEQ ID NO 1 or a sequence with at least a 35% similarity with the SEQ ID NO 1;
b. identifying the transformed organisms in a) through selection with antibiotic,
c. seeding the salinized medium with organisms identified in b),
d. cultivating the organisms during an adequate period, and
e. collecting the organisms

13. Use of phytochelatines obtained *in vivo or in Vitro* by enzymatic reaction mediated by phytochelatine synthase codified by the SEQ ID NO 1, or sequences with at least a 35% similarity, as sodium chelants.
